# FASCICULE DE BREVET EUROPEEN

(11) **EP 1 589 942 B1**
(45) Date de publication et mention de la délivrance du brevet: **03.01.2007**
(21) Numéro de dépôt: 04707553.6
(22) Date de dépôt: 03.02.2004
(51) Int. Cl.: A61Q 19/00, A61K 8/92

(54) **PROCEDE D'EXTRACTION DE LIPIDES A PARTIR DE MOLLUSQUES NACRIERS**
VERFAHREN ZUM EXTRAHIEREN VON LIPIDEN AUS PERLMOLLUSKEN
METHOD FOR THE EXTRACTION OF LIPIDS FROM NACREOUS MOLLUSCS

(30) Priorité: 04.02.2003 FR 0301246
(43) Date de publication de la demande: 02.11.2005
(73) Titulaire: Robert Wan Holding, 98713 Papeete-Tahiti (FR)
(72) Inventeur: LOPEZ, Evelyne, F-75007 Paris (FR); ROUSSEAU, Marthe, F-29200 Brest (FR)
(74) Mandataire: Fruchard, Guy
(86) Numéro de dépôt international: PCT/FR2004/000240
(87) Numéro de publication internationale: WO 2004/078156

(56) Documents cités:
- FR-A- 2 799 125
- DUNSTAN ET AL.: "The effect of lyophilization on the solvent extraction of lipid classes, fatty acids and sterols from the oyster crassostrea gigas" LIPIDS, vol. 28, no. 10, 1993, pages 937-944, XP0008023254 USA
- PATENT ABSTRACTS OF JAPAN vol. 1996, no. 11, 29 novembre 1996 (1996-11-29) & JP 8 175950 A (MIKIMOTO PHARMACEUT CO LTD), 9 juillet 1996 (1996-07-09)
- DATABASE WPI Section Ch, Week 199032 Derwent Publications Ltd., London, GB; Class D21, AN 1990-242898 XP002257518 & JP 02 169509 A (MIKIMOTO SEIYAKU KK) 29 juin 1990 (1990-06-29)

## Description

La présente invention concerne un procédé d'extraction d'un principe actif provenant de mollusques nacriers.

### ARRIERE PLAN DE L'INVENTION

On sait, notamment du document FR-A-1 350 038, et du document JP-A-8175950 que pour régénérer la barrière cutanée de la peau, il est souhaitable de traiter celle-ci avec des compositions cosmétiques régénérantes contenant un principe actif riche en lipides, notamment en lipides polaires extraits de la chair de mollusques nacriers, en particulier de la chair d'huître. Le traitement d'extraction est complexe car il est tout d'abord nécessaire de broyer la chair soit après l'avoir séchée, soit après l'avoir déshydratée. Le traitement est donc coûteux. En outre, le pourcentage de produit actif dans l'extrait obtenu est très faible de sorte que le prix du produit actif est très élevé.

### OBJET DE L'INVENTION

Un but de l'invention est de proposer un procédé permettant d'extraire un principe actif extrait d'un mollusque nacrier avec de meilleures performances qu'avec les procédés existants.

### DESCRIPTION DETAILLEE DE L'INVENTION

On propose un procédé d'extraction d'un principe actif provenant de mollusques nacriers comportant les étapes de séparer la nacre du reste de la coquille d'un mollusque nacrier, de broyer la nacre selon une poudre fine, de préférence ayant une granulométrie moyenne inférieure à 20 µm et plus particulièrement une granulométrie moyenne de l'ordre de 8 µm, de procéder à une extraction lipidique à partir de la poudre obtenue en soumettant celle-ci à un solvant lipidique, puis d'extraire le principe actif à partir du solvant.

L'extraction lipidique peut être réalisée par tout procédé connu tel que par exemple en soumettant la poudre à un solvant lipidique tel qu'un mélange de chloroforme et de méthanol, ou de l'éthanol chaud puis en effectuant une séparation par centrifugation pour séparer une phase solide d'une phase liquide, suivie d'une élimination du solvant de la phase liquide par l'évaporation. Le principe actif obtenu se présente sous forme d'un gel brun qui peut être directement utilisé dans la composition cosmétique selon l'invention. En pratique, en utilisant une poudre de nacre ayant une granulométrie moyenne de l'ordre de 8 µm, on obtient environ cinq kilogrammes de principe actif à partir d'une tonne de nacre. On notera à ce propos que la nacre est un solide facilement broyable de sorte que le traitement est peu coûteux. En outre la coquille d'huître est un déchet pratiquement sans valeur de sorte que l'investissement pour l'achat de la matière première peut être considéré comme nul.

Le principe actif ainsi obtenu est mélangé avec un support cosmétique connu en soi dans une proportion d'au moins 0,2 %, et de préférence 0,5 à 1 % en volume ou en poids selon les applications envisagées.

Bien entendu, l'invention n'est pas limitée au mode de réalisation décrit ci-dessus et est susceptible de variantes de réalisation sans sortir du cadre de l'invention tel que défini par les revendications.

En particulier, bien qu'il ait été prévu de broyer la nacre selon une granulométrie moyenne de l'ordre de 8 µm, on peut réaliser un broyage moins fin, selon une granulométrie moyenne de préférence inférieure à 20 µm, le rendement d'extraction étant d'autant plus faible que le broyage est plus grossier.

Bien que l'invention ait été décrite en utilisant de la nacre ayant pour origine une huître du genre Pinctada espèce margaritifera, on peut non seulement utiliser des huîtres d'un genre ou d'une espèce différente, mais également utiliser d'autres mollusques nacriers.

On peut également procéder à une extraction lipidique à partir d'un mélange de poudre provenant du corps et de la nacre d'un même mollusque ou de mollusques différents les uns des autres, en particulier lorsqu'une analyse des principes actifs obtenus révèle des différences rendant utile la réalisation d'une combinaison.

## Revendications

1. Procédé d'extraction d'un principe actif lipidique à partir d'une matière solide provenant d'un mollusque nacrier, **caractérisé en ce qu'**il comporte les étapes de broyer de la nacre selon une poudre fine, de mettre la poudre en contact avec un solvant lipidique, de séparer le solvant et d'extraire le principe actif lipidique par évaporation du solvant.

2. Procédé selon la revendication 1, **caractérisé en ce que** la nacre est broyée selon une poudre ayant une granulométrie moyenne inférieure à 20 µm.

3. Procédé d'extraction de lipides selon la revendication 2, **caractérisé en ce que** la NACRE est broyée selon une poudre ayant une granulométrie moyenne de l'ordre de 8 µm.

4. Procédé selon la revendication 1, **caractérisé en ce que** la nacre provient d'huîtres du genre Pinctada espèce margaritifera.

## Claims

1. A method of extracting a lipidic active principle from a solid material coming from a nacreous mollusk, the method being **characterized in that** it comprises the steps of grinding the nacre into a fine powder, of putting the powder into contact with a lipid solvent, of separating the solvent, and of extracting the lipid active principle by evaporating the solvent.

2. A method according to claim 1, **characterized in that** the nacre is ground to a powder having a mean grain size of less than 20 µm.

3. A method of extracting lipids according to claim 2, **characterized in that** the nacre is ground to a powder having a mean grain size of about 8 µm.

4. A method according to claim 1, **characterized in that** the nacre comes from oysters of the genus Pinctada species margaritifera.

## Patentansprüche

1. Verfahren zum Extrahieren eines lipidischen Wirkstoffes aus einem Feststoff, der aus einem Perlmutt bildenden Mollusken stammt, **dadurch gekennzeichnet, dass** es die Schritte umfasst: Zermahlen des Perlmutts zu einem feinen Pulver, Kontaktieren des Pulvers mit einem Lipidlösungsmittel, Abscheiden des Lösungsmittels und Extrahieren des Lipids durch Verdampfung des Lösungsmittels.

2. Verfahren nach Anspruch 1, **dadurch gekennzeichnet, dass** das Perlmutt zu einem Pulver mit einer mittleren Korngröße von unter 20 µm zermahlen wird.

3. Verfahren zum Extrahieren von Lipiden nach Anspruch 2, **dadurch gekennzeichnet, dass** das Perlmutt zu einem Pulver mit einer mittleren Korngröße in der Größenordnung von 8 µm zermahlen wird.

4. Verfahren nach Anspruch 1, **dadurch gekennzeichnet, dass** das Perlmutt von Austern der Art Pinctada Margaritifera stammt.
